# EUROPEAN PATENT SPECIFICATION

(11) **EP 3 856 046 B1**
(45) Date of publication and mention of the grant of the patent: **28.10.2026**
(21) Application number: 19770151.9
(22) Date of filing: 11.09.2019
(51) Int. Cl.: A61B 17/12, A61B 17/00

(54) **SURGICAL DEVICE**
CHIRURGISCHE VORRICHTUNG
DISPOSITIF CHIRURGICAL

(30) Priority: 25.09.2018 GB 201815630
(43) Date of publication of application: 04.08.2021
(73) Proprietor: Vascutek Limited, Renfrewshire PA4 9RR (GB)
(72) Inventor: NIMMO, Seonaid, Barbara, Glasgow, Strathclyde G20 6UN (GB)
(74) Representative: Murgitroyd & Company
(86) International application number: PCT/GB2019/052533
(87) International publication number: WO 2020/065260

(56) References cited:
- WO-A1-01/76487
- WO-A1-97/01309
- DE-A1- 2 402 677
- US-A1- 2005 256 365

## Description

### Field of Invention

This invention relates to surgical devices for controlling fluid flow through a vessel defining a lumen. Natural vessels defining a lumen enabling fluid flow in physiological functioning systems and organs include tubular connective tissues of the vasculature, lymphatic system, renal system, respiratory system etc. Synthetic or prosthetic devices also include tubular parts through which control of fluid flow is desirable during at least part of a surgical procedure. The present disclosure is applicable to both natural and synthetic vessels having a length and a transverse dimension defining a lumen or through passage, which vessels may be flexible walled vessels which can be closed to through-flow by a clamp for example.

### Background of Invention

Commercially available valves for providing haemostasis in endovascular delivery systems are considered by many to be unduly expensive and tend to be over-engineered. Over time these valves have been found to lose their efficacy and the ability to provide haemostasis for example is lost.

Surgical sutures, loops or ligatures are currently used for constricting or ligating natural vessels during surgery. An arrangement comprising a wire-like surgical loop with a holding member is disclosed in WO 01/76487. However to loosen and tighten these surgical loops has been found to be awkward and time consuming during a surgical procedure, when efficient use of time is crucial.

Other devices for controlling fluid flow through a lumen of a natural vessel are described in the following documents: an implantable mechanical sphincter for controlling urinary incontinence is described in WO 97/01309 A1, said sphincter comprising a springy semi-annular piece in whose free ends are affixed two parallel strips made of porous anti-adherent material. When implanted in the patient's urethra the strips surround and exert permanent pressure around said urethra, thus avoiding the involuntary emission of urine. DE 2402677 A describes a surgical blood-vessel clamp which has an elastic sleeve with a hole bounded by an inner wall, to receive the ends of an elongate flexible ligature that forms a loop about the section of the vessel to be shut off. US 2005/256365 describes a penile compression device with semi-rigid first and second support arms connected at opposing ends of their length direction. These arms are biased toward each other in a resting (closed) position, but are manually compressible from the length direction causing shape deformation to form an open region between the support arms for penis insertion. Releasing clamp compression allows the support arms to bias to a clamp compressing the male urethra to prevent urinary leakage. There remains a need for a device for use during a surgical procedure, when efficient use of time is crucial.

It is an object of the present invention to mitigate or obviate at least one problem with prior devices and provide a device which is simple in design and easy to use.

### Summary of Invention

According to the present invention, there is provided a device for controlling fluid flow through a vessel defining a lumen through which fluid may flow, as defined in the independent claims, with dependent claims relating to embodiments.

In embodiments, the operating member may be formed from a flexible material with sufficient resilience to provide tension in the at least one elongate flexible member when required, whilst allowing a user to manipulate the operating member to reduce the tension in the at least one elongate flexible member when required. The common point of the operating member may be a flexure to provide for relative movement of the first and second arms of the operating member or a spring biased hinge portion. The material selected for the operating member, particularly the flexure provides for recoverable strain without permanent deformation.

In embodiments where the or each elongate member is selected from materials known to be relatively highly flexible, for example silicone rubber cord, the person skilled in the art will understand and give due consideration to material parameters for the elongate flexible member including tensile modulus (in GPa) and recoverable tensile strain (%). Both of these properties can be measured for the elongate flexible member using a uniaxial tensile stress-strain test (e.g. according to ASTM D 412, ASTM D638, or optionally ASTM D1708), where the elongate member sample is loaded in tension to a certain maximum deflection and unloaded. In this case, typical suitable modulus range for the 'flexible' elongate member would be from 0.01 to 0.1 GPa and typical suitable recoverable strain would be from 5% to 300%. However, these are only example ranges, and further embodiments are contemplated where the elongate flexible member has properties outside this range (for example, an elongate member of material with higher modulus and lower recoverable strain but with different cross-sectional / length geometry). In this case, embodiments are also contemplated where the elongate flexible member comprises a polymer or metal / alloy wire or braid.

In this case using such a flexible elongate member with relatively low tensile stiffness and high recoverable strain properties means that the 'eye' can be enlarged by extension of the elongate member material with relatively low force (for example, by insertion of a larger catheter over a guidewire within the vessel, while maintaining haemostasis), and that this extension will not result in permanent deformation of the elongate member.

For the case where the elongate member is chosen to be relatively stiff in tension (e.g. monofilament or multifilament suture, nylon or polyethylene based braided string) the key material parameter is tensile stiffness. In this case, the stiffness should be sufficiently high that there is negligible extension of the elongate member under the forces involved for achieving the haemostatic seal. In terms of the materials used in this case, they may be polymers with Young's modulus in the range of 1 to 10 GPa. However, other materials with a different modulus could be chosen to give an elongate member design with sufficient tensile stiffness.

In this case, the elongate member's relative tensile stiffness means that the size of the 'eye' is controlled in use primarily by the position of the compressible operating member (i.e. compression upon the arms enables a user to enlarge the eye, and release of the arms allows the elongate flexible members to recover and thereby to reduce eye diameter).

In an embodiment, the compressible operating member is attachable to the first attachment point and the second attachment point of said at least one elongate flexible member, at least one arm of the compressible operating member being compressible upon the application of pressure to the compressible operating member, and operable to act upon the at least one elongate flexible member such that: the at least one elongate flexible member is movable from a first position in which the at least one elongate flexible member is under tension such that the eye constricts fluid flow within the lumen, and a second position in which the at least one elongate flexible member is relaxed such that the eye does not constrict fluid flow through the lumen.

The application of pressure to the compressible operating member may be that of finger pressure of a user of the device. The device is conveniently hand-held by a user, and pressure may be applied by compression of the compressible operating member of the device between a thumb and one or more fingers of the user.

The compressible operating member may comprise first and second arms respectively extending from a common point such as a hinge, joint, or flexible portion such that the compressible operating member may be configured as substantially C-shaped, V-shaped or U-shaped, or the like, with spaced apart free ends which are movable one towards the other when compressed by a user. The first and second arms may individually have straight or curved length parts. The first and second arms may be made from a resilient form-retaining material such that when a user releases the first and second arms from a compressed state, the first and second arms return to their original configuration. The uncompressed state may be considered as a first configuration wherein the elongate flexible member is in a first position. The compressed state may be considered as a second configuration wherein the elongate flexible member is in a second position.

The device may incorporate a configuration biasing member to ensure that the first and second arms return to the uncompressed first configuration after a user has compressed and released the compressible operating member. The configuration biasing member may be a spring such as a coil spring, leaf spring, torsion spring or the like arranged between the first and second arms in a space of sufficient width to avoid interference with the at least one elongate flexible member, optionally at or near the hinge, joint, or flexible portion of the compressible operating member to bias the first and second arms towards the uncompressed first configuration. The use of such a configuration biasing member may also be beneficial in maintaining or countering tension in the at least one elongate flexible member.

In embodiments where the operating member is of a less flexible or of a stiffer material which is more resistant to compression by hand or finger pressure of a user, the at least one elongate flexible member may be an elongate resilient member retaining elastic properties under tension. The elongate resilient member would exhibit limited elongation under tensile loads, but return to its original length when loading is removed. Whilst under tensile load the elongate resilient member would exhibit resistance to further elongation or "creep".

Whereas natural fibers such as silk or cotton may be used alone or coated to modify the natural properties thereof, a flexible silicone material may be suitable for the at least one elongate flexible member. The at least one elongate flexible member may be a synthetic filament which filament may be optionally silicone-coated, or at least partially PTFE-coated, which filament may be based on a nylon, a polyester, polypropylene, polyvinylidene fluoride (PVDF) or another flexible material of similar physical properties that is easily manipulated and shaped but is not permanently stretched under tension as contemplated for the disclosed use when attached to the compressible operating member.

The at least one elongate flexible member is intended to provide a readily manipulated mechanism that is user-initiated to shift from a lumen occluded condition to a lumen open condition, but it will be understood that a user may grip and manipulate the elongate flexible member through positions intermediate the first position and second position.

The "vessel" may be a natural vessel such as a blood vessel. Alternatively, the "vessel" may be a synthetic vessel such as a branch of a vascular graft. The "elongate flexible member" should be understood to be an elongate flexible member capable of being readily formed into at least one loop to define an internal space within the loop which is referred to herein as "an eye". The elongate flexible member may be for example a strand, thread, filament, ribbon, fibre or cord, or a piece of string, the internal space within the loop being adapted to receive the width of the vessel. The elongate flexible member may be easily manipulated and shaped, and resilient but may not be stretched under tension beyond the elastic limits of the material from which the elongate flexible member is made in the disclosed use when attached to the compressible operating member.

In the case of application to a prosthetic device having soft fabric tubular portion, it will be understood that closure of the eye around the soft fabric tubular portion of the prosthetic device permits a conformable seal around any object presented within the soft fabric tubular portion of the prosthetic device. Thus it is thereby possible to provide a haemostatic seal, whilst permitting passage of any object such as a catheter, wire, or other typical delivery system component through the soft fabric tubular portion of the prosthetic device if required. In this way the disclosed device acts as a readily applied and removable haemostatic valve. Optionally the device may be tethered to the graft to keep it in position during the surgical procedure.

The operating member may be operable to act on the at least one elongate flexible member such that the at least one elongate flexible member is biased towards the first position.

The operating member may be a bifurcated device such as a "C"-shaped member, or V-shaped or U-shaped, or similar configuration having first and second arms separated by a gap. The first arm of the operating member may be attachable to the first attachment point of the at least one elongate flexible member, and the second arm of the operating member may be attachable to the second attachment point of the at least one elongate flexible member. The at least one elongate flexible member may be manipulated to form at least one loop defining the eye before attachment to the operating member. The eye may be located in the gap between the first and second arms of the operating member. At least one of the first and second arms of the operating member may be movable inwardly relative to the other of the first and second arms of the operating member so as to move the at least one elongate flexible member from the first position to the second position. The first and second arms may each have an aperture through which the at least one elongate flexible member may be passed and attached to the respective arm in an assembly step prior to use. The aperture respectively in each of the first and second arms may be accessible from above and below the arm, i.e. an aperture which is circular or oval, or additionally accessible through a side slot, for example an aperture which when viewed from above the arm is substantially J-shaped, or substantially L-shaped. The apertures may form part of an attachment structure for example to serve as a threading arrangement for attachment of the at least one elongate flexible member.

In an embodiment at least one aperture through which the at least one elongate flexible member may be passed may be a pinhole aperture in at least one of the first and second arms of the operating member. The pinhole aperture may be defined by a surface configured such that at least a portion of the at least one elongate flexible member interacts with the surface upon contact therewith such that friction between the surface and the contacting portion of the at least one elongate flexible member acts to locate the at least one elongate flexible member sufficiently without additional fastening means. The pinhole aperture-defining surface may be roughened, ribbed, grooved, or serrated to increase frictional interaction with the at least one elongate flexible member. Thereby the at least one elongate flexible member can be suitably positioned and configured for use, yet avoid undesirable loosening or slackening in use. Preferably each of the first and second arms is provided with such a pinhole aperture. A user can selectively alter the configuration, for example to enlarge the "eye" by applying sufficient force to a portion of the at least one elongate flexible member to overcome friction between the contacting portion of the at least one elongate flexible member and the pinhole aperture-defining surface.

In an embodiment the operating member and the at least one elongate flexible member are pre-assembled to provide a user with a "ready for use" device for controlling fluid flow through a vessel defining a lumen through which fluid may flow. In such an embodiment the at least one elongate flexible member is threaded through the apertures and pulled through by a length sufficient to form a loop defining an eye of a desired size.

The at least one elongate flexible member may be made from a material which has a reduced tendency to slip through the aperture absent a user applied pull of sufficient force to overcome friction between the contacting portion of the at least one elongate flexible member and the pinhole aperture-defining surface. A flexible silicone material may be suitable for the at least one elongate flexible member. At least a portion of the flexible silicone material may be covered with a removable low-friction material to facilitate assembly when threading the at least one elongate flexible member through the aperture(s). Polytetrafluoroethylene (PTFE) tape may serve as the removable low-friction material. Monofilament or multi-filament braided materials may be used for the at least one elongate flexible member.

The at least one elongate flexible member may be a single elongate flexible member comprising first and second ends. The first arm of the operating member may comprise a first threading arrangement, and the second arm of the operating member may comprise a second threading arrangement. The first end may be threaded through the first threading arrangement, and the second end may be threaded through the second threading arrangement.

Each of the first and second threading arrangements may comprise an aperture which is shaped so as to provide a plurality of winding points for the thread.

At least one, and optionally both of the first and second threading arrangements may be a transversely arranged J-slot accessible from a side of the arm of the operating member.

The at least one elongate flexible member may comprise: a first elongate flexible member configured to form a first loop; and a second elongate flexible member configured to form a second loop. The first and second elongate flexible members may be configured such that the first and second loops are juxtaposed so as to define the eye for surrounding the lumen. For example the first and second loops may be linked together. The first elongate flexible member may comprise the first attachment point and the second elongate flexible member may comprise the second attachment point.

The first arm of the operating member may comprise a first threading arrangement, and the second arm of the operating member may comprise a second threading arrangement. The first elongate flexible member may be threaded through the first threading arrangement and the second elongate flexible member may be threaded through the second threading arrangement.

Each threading arrangement may comprise an aperture, and each elongate flexible member may extend through the aperture through the loop formed by the other elongate flexible member and back through the aperture. The aperture of each threading arrangement may be defined by a surface configured such that at least a portion of the at least one elongate flexible member interacts with the surface upon contact therewith such that friction between the surface and the contacting portion of the at least one elongate flexible member acts to locate the at least one elongate flexible member sufficiently without additional fastening or retention means. The aperture-defining surface may be roughened, ribbed, grooved, or serrated to increase frictional interaction with the at least one elongate flexible member. Thereby the at least one elongate flexible member can be suitably positioned and configured for use, yet avoid undesirable loosening or slackening in use.

The threading of the at least one elongate flexible member for attachment of the at least one elongate flexible member to the first and second arms of the operating member may be secured by tying a knot in the at least one elongate flexible member to prevent slippage through the aperture in the arm of the operating member in use.

The at least one elongate flexible member may be formed from a silicone.

The at least one elongate flexible member may be a resilient cord.

The operating member may be compressible upon the application of finger pressure.

In use of the device, especially in embodiments where the at least one elongate flexible member is in fact a single elongate flexible member, the maintenance of the eye in a dilated loop configuration whilst manipulating the device with respect to application to, or removal from, a vessel may be facilitated by insertion of an elongate keeper which may be any elongate instrument that is to hand such as a thin probe, end of a catheter, handle of a scalpel, urethral dilator, or the like.

In preferred embodiments the use of two intersecting loops of first and second elongate flexible members operable together to define an eye of variable dimensions provides that the formed eye remains available for dilation or closing about a vessel at all times.

### Brief Description of the Drawings

Preferred embodiments of the present invention will now be described, by way of example only, with reference to the accompanying drawings, in which:
Figure 1 shows a device according to a first embodiment of the present invention;
Figures 2A, 2B, and 2C show a device according to a second embodiment of the present invention;
Figure 3A shows a perspective view of a compressible operating member of generally C-shape which forms part of a manually controlled fluid flow device in an embodiment of the invention;
Figure 3B shows a longitudinal side section through the compressible operating member shown in Figure 3A and illustrating upper surface and lower surface exterior indentations for a user's finger to facilitate compression of the operating member in use;
Figure 3C shows a longitudinal side section through the compressible operating member shown in Figure 3A from above and to one side to illustrate more clearly the ribbed inner surface of each threading aperture for receiving an elongate flexible member;
Figure 4 shows an embodiment of a device of the present invention assembled for use and comprising the compressible operating member with dual intersecting threaded elongate flexible members defining an eye for receiving a vessel (not shown) to be constricted during a surgical procedure; and
Figure 5 shows a device according to the second embodiment in use upon a branch vessel of tubular prosthetic implant.

### Detailed Description of the Drawings

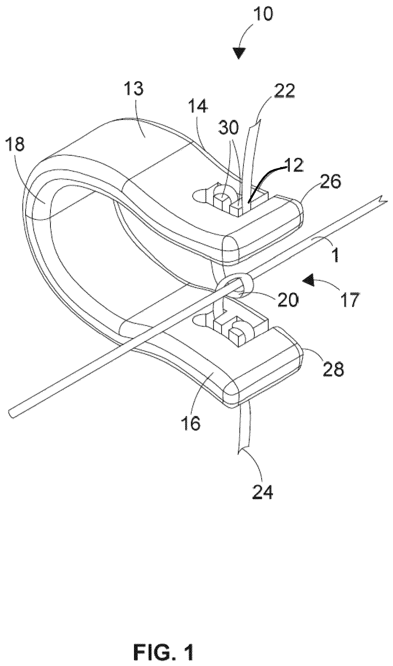

Reference is now made to Figure 1, which shows a device **10** for controlling fluid through a vessel 1 defining a lumen, in accordance with a first embodiment of the present invention. The device 10 comprises an elongate flexible member **12,** which in the depicted example is a flexible cord.

The device 10 also comprises a compressible operating member **13,** which a C-shaped member. The C-shaped member 13 comprises first and second arms **14, 16,** having distal free ends, which are separated by a gap **17** and extend from a common point in the form of a proximal hinge portion **18** of the C-shaped member. In the depicted example, the hinge portion 18 is a flexible U-shaped portion of the C-shaped member 13. As shown in Fig. 1, the elongate flexible member 12 is configured to form an eye **20** for surrounding the vessel 1. The eye 20 is located in the gap 17 and in use may lie substantially transversely over the exterior of the vessel 1.

The terms "hinge" and "hinge portion" as used in this disclosure and claims relates to a flexible connection, joint, or return portion between proximal ends of the first and second arms of this or any embodiment whereby at least one of the first and second arms can be moved towards the other of the first and second arms.

The first arm 14 comprises a first threading arrangement **26,** and the second arm 16 comprises a second threading arrangement **28.** The elongate flexible member 12 is attached to the C-shaped member 13 by threading a first end **22** of the elongate flexible member through the first threading arrangement 26 and by threading a second end **24** of the elongate flexible member through the second threading arrangement 28.

In the depicted example, each of the first and second threading arrangements 26, 28 is an aperture which comprises a comb-shaped portion. Each aperture therefore forms a plurality of teeth **30** of the C-shaped member 13. In the depicted example, each end of the elongate flexible member 12 is threaded around the teeth 30 of its associated threading arrangement 26, 28. Each portion of the elongate flexible member 12 located in one of the threading arrangements 26, 28 forms a friction fit with that threading arrangement, which allows the portion of the first elongate flexible member 12 located in the gap 17 to be tensioned, and the eye to be biased towards a closed position. The elongate flexible member 12 is threaded through the threading arrangements 26, 28 such that in a rest position the portion of the elongate flexible member 12 located in the gap 17 is under tension and the eye 20 may constrict fluid flow through a lumen.

Either of the arms 14, 16 is movable inwardly in response to pressure applied to its outer surface. This means that if pressure is applied to one of the arms 14, 16, the portion of the elongate flexible member located in the gap 17 is relaxed, which has the effect of increasing the size of the eye 20, and allowing fluid flow through the lumen. Each of the arms 14, 16 is preferably movable in response to finger pressure applied thereto.

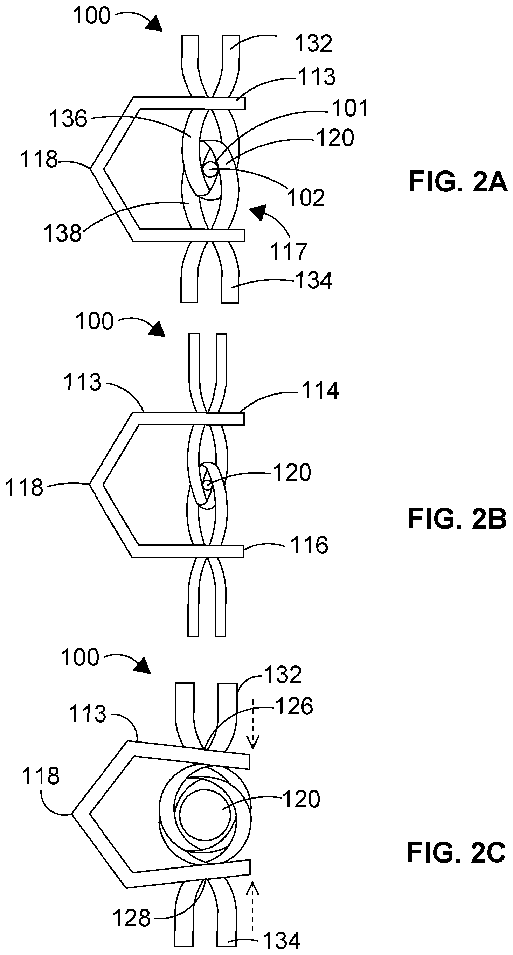

Reference is now made to Figures 2A, 2B and 2C, which show a device 100 for controlling fluid flow through a vessel **101** defining a lumen **102,** in accordance with a second embodiment of the present invention. In the embodiment depicted, the device 100 comprises first and second elongate flexible members **132, 134.** The first elongate flexible member 132 is configured to form a first loop **136,** and the second elongate flexible member 134 is configured to form a second loop **138.** As shown, the first loop 136 is linked with the second loop 138 so as to define an eye **120** for surrounding the vessel 101. The use of first and second elongate flexible members 132, 134 configured such that a portion of one of said first and second elongate flexible members 132, 134 passes through the loop of the other at least once to form intersecting loops, provides stability to the eye 120 so that the eye configuration is preserved even if an object such as a vessel or catheter, or surgical implement is not present within the eye. A stabilised eye is easier to use, for example, when a user wants to pass the eye over an object or insert an object into the eye. The use of intersecting loops is more advantageous than use of a single loop which may loosen and uncoil if not looped around an object.

The device 100 also comprises an operating member **113** which is operable to act upon the first and second elongate flexible members 132, 134 such that the eye 120 is reclosable. The operating member 113 in this embodiment is a C-shaped member.

The operating member 113 has first and second arms **114, 116,** which are separated by a gap **117** where the eye 120 is located. The first and second arms 114, 116 extend upwardly from a hinge portion 118.

Each arm 114, 116 comprises a threading arrangement **126, 128** for each elongate flexible member 132, 134. In the depicted example, each threading arrangement 126, 128 comprises a single aperture. In the depicted example, each elongate flexible member 132, 134 is threaded through its respective aperture 126, 128 into the gap 117, through the loop of the other elongate flexible member and back through its respective aperture. Each elongate flexible member 132, 134 may be a resilient cord, and sized relative to the size of its respective aperture 126, 128 such that it forms a friction fit with its respective arm inside its respective aperture. This allows the portion of each elongate flexible member 132, 134 forming the loop to be tensioned by adjusting the size of the loop.

In this embodiment both loops 136, 138 are linked with each other so that they cooperate, which has the overall effect of stabilising the eye and under tensile biasing by the operating member 113 the loops 136, 138 tend towards a rest position in which the eye 120 is closed, as shown in Figure 2B. As shown in Figure 2C, the arms 114, 116 are movable inwardly. This means that when a sufficient compressive force is applied to the operating member 113 such that the arms move inwardly, each of the loops 136, 138 slacken, thereby opening the eye 120.

The use of a device as disclosed herein will now be described with reference to the first embodiment described above with reference to Figure 1. In use, the device 10 of the first embodiment may be provided such that the eye 20 already surrounds the vessel 1, if the vessel 1 is a branch of a vascular graft for example. Alternatively, the device 10 may be assembled *in situ* by a medical professional, as described below. When assembling the device 10, the medical professional configures the elongate flexible member 12 such that it forms the eye 20 with the vessel 1 located therein. As part of the assembly procedure, the medical professional threads the first end 22 of the elongate flexible member 12 through the first threading arrangement 26, and threads the second end 24 through the second threading arrangement 28. The medical professional then tensions the portion of the elongate flexible member 12 located in the gap 17 such that the eye constricts flow through the vessel 1. If the medical professional wishes to allow fluid through the vessel 1, the medical professional may grip the operating member and manipulate it between fingers and thumb such that simple application of finger pressure to the operating member 13, has the effect of altering the dimensions of the eye 20, changing the eye 20 into a configuration where the eye 20 opens to allow fluid flow through the vessel. As the operating member 13 biases the eye 20 towards a closed position, when the medical professional removes this finger pressure, the eye 20 returns to a tighter configuration around the vessel 1, whereby the eye 20 constricts the vessel 1 to inhibits fluid flow through the vessel.

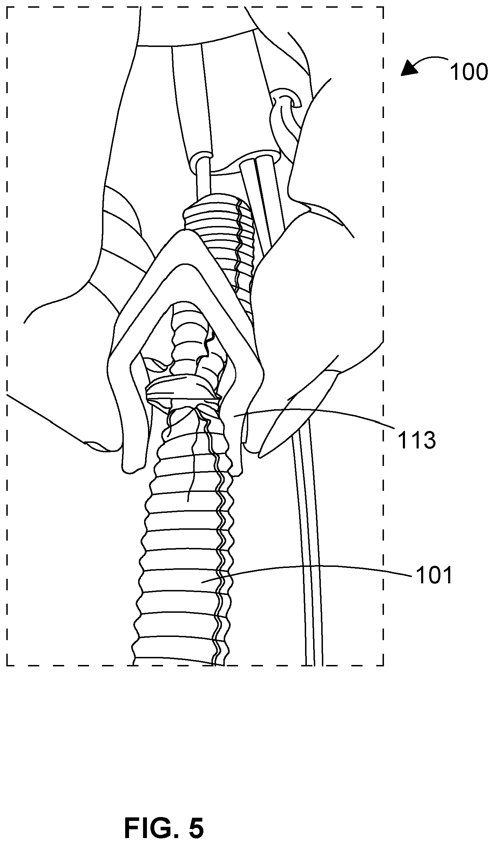

The use of the second embodiment will now be described with reference to Figs. 2A, 2B and 2C.In use, the device 100 of the second embodiment may be provided such that the eye 120 already surrounds the vessel 101, if the vessel is a tubular branch of a prosthetic device such as a vascular graft for example. Alternatively, the device 100 may be assembled *in situ* by a medical professional, as described below. When assembling the device 100, the medical professional configures the first and second elongate flexible members 132, 134 such that they each form a loop and are linked so as to define the eye 120. The medical professional may do this by threading each elongate flexible member 132, 134 through its respective aperture 126, 128 into the gap 117, and one of the elongate flexible members 132, 134 through the loop of the other elongate flexible member and back through its respective aperture 126 or 128. The medical professional then tensions each of the elongate flexible members such that the eye constricts fluid flow through the vessel. With reference to Figure 5, when the medical professional wishes to allow fluid flow through the vessel, the medical professional may grip the operating member and manipulate it such that simple application of pressure to the operating member 113, has the effect of moving the eye into a position where it allows fluid flow through the vessel 101. As the operating member 113 biases the eye towards a closed position, when the medical professional removes this pressure, the eye returns to a position where it prevents fluid flow through the vessel 101.

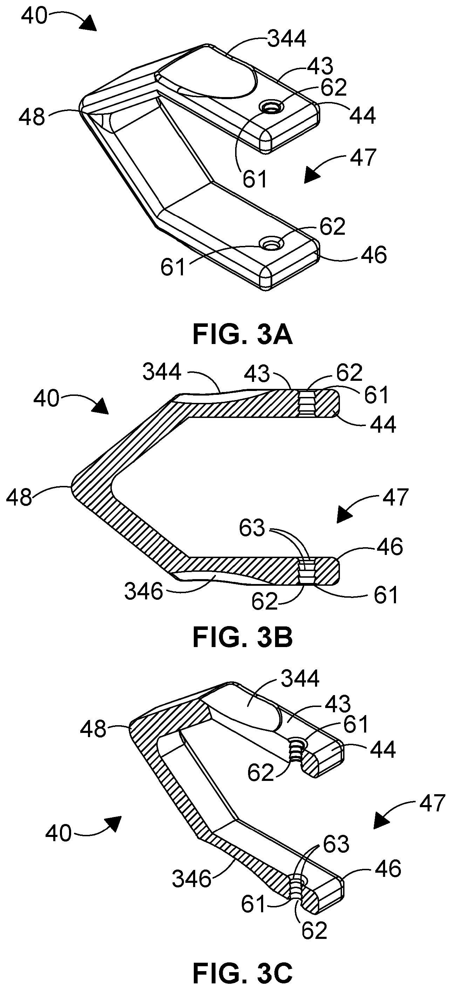

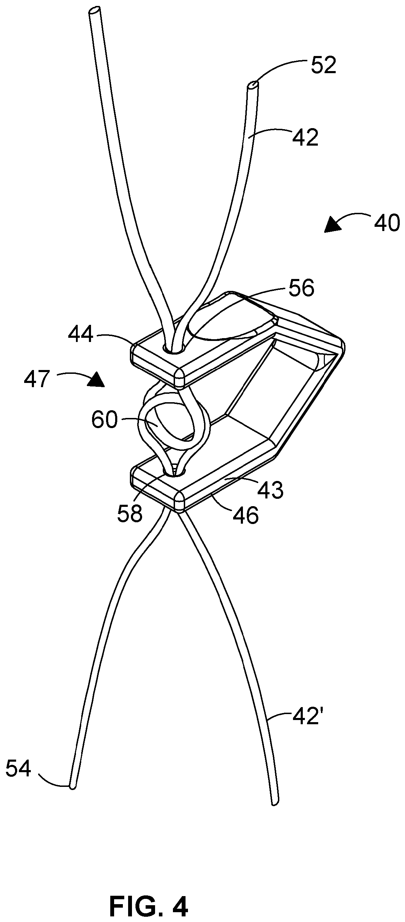

A further embodiment which is commercially attractive due to its relative lack of complexity, and which offers an advantage of predictable reliability in use, will now be described referring to Figs. 3A, 3B, and 3C, together with Fig. 4.

Reference is first made to Figure 4, which shows an assembled device **40** suitable for controlling fluid through a vessel (not shown) defining a lumen, in accordance with another embodiment. The device 40 comprises an elongate flexible member **42,** which in the depicted example is formed from two intersecting threaded elongate flexible members 42, 42' operable together to define an eye **60** of variable dimensions. Each of the elongate flexible members 42, 42' is made from a flexible silicone cord. The elongate flexible members 42, 42' are respectively threaded through arms of a user-compressible operating member 43 illustrated in isolation in Figs. 3A, 3B, and 3C.

Referring to Figs. 3A, 3B, and 3C the user compressible operating member 43, in this embodiment is a generally C-shaped member with a proximal hinge portion **48** and first and second arms **44, 46** extending from the proximal hinge portion 48 and having free distal ends. The distal ends of the first and second arms 44, 46 are separated by a gap **47.** In the depicted example, the hinge portion 48 is a V- or U-shaped portion of the C-shaped member 43. Each of the first and second arms 44, 46 has a pinhole aperture **61** near the distal end of the respective first and second arms 44, 46. An inner surface **62** defining each pinhole aperture 61 is ribbed in this embodiment to frictionally interact with a portion of the elongate flexible member 42, 42' when threaded through the respective pinhole 61.

As shown in Fig. 4, the compressible operating member 43 receives a pair of elongate flexible members 42, 42', which together function as one to form the elongate flexible member which is configured to form an eye **60** for surrounding the vessel. The eye 60 is located in the gap 47.

The first arm 44 comprises a first threading arrangement **56,** and the second arm 46 comprises a second threading arrangement **58.** The elongate flexible member 42, 42' is attached to the C-shaped member 43 by threading a first end **52** of the elongate flexible member 42 through the first threading arrangement 56 to form a first loop, and then returning the first end 52 back through the first threading arrangement 56. Then the second elongate flexible member 42' has a free end defined here as the second end **54** that is threaded through the second threading arrangement 58, through the first loop to form a linked second loop and then returning the second end 54 back through the second threading arrangement 58.

In the depicted example, referring again to Figs. 3A, 3B and 3C each of the first and second threading arrangements 56, 58 is configured as a pinhole aperture 61 which comprises a ribbed inner surface portion 62. Each pinhole aperture therefore forms a plurality of ribs **63** within the pinhole aperture 61 of the C-shaped member 43. In the depicted example, each end of the elongate flexible member 42, 42' is threaded past the ribs 63 of its associated threading arrangement 56, 58. Each portion of the elongate flexible member 42, 42' located in one of the threading arrangements 56, 58 forms a friction fit with the ribbed surface portion 62 within the pinhole aperture 61 of the threading arrangement 56, 58, which allows a portion of the first elongate flexible member 42, 42' located in the gap 47 to be tensioned, and the eye 60 to be biased towards a closed position. The elongate flexible member 42, 42' is threaded through the pinhole aperture 61 of each of the threading arrangements 56, 58 such that in a rest position the portion of the elongate flexible member 42 located in the gap 47 is under tension and the eye 60 may constrict fluid flow through a lumen of a vessel.

Each of the arms 44, 46 is movable inwardly in response to pressure applied to its outer surface. This means that if pressure is applied to one of the arms 44, 46, the portion of the elongate flexible member 42 located in the gap 47 is relaxed, which has the effect of increasing the size of the eye 60, and allowing fluid flow through the lumen. Each of the arms 44, 46 is preferably movable in response to finger pressure applied thereto. An indentation **344, 346** in an outer surface of the respective arms 44, 46 serves as a finger-pressure guide for a user's finger to be applied with reduced risk of slippage off the arm during use of the device 40 in a surgical procedure where body fluids may wet device surfaces thereby interfering with intended use of the device 40.

The at least one elongate flexible member may be formed from a flexible silicone material or a filament which material or filament may be optionally silicone-coated, or at least partially PTFE-coated, which filament may be based on a nylon, a polyester, polypropylene, polyvinylidene fluoride (PVDF) or another flexible material of similar physical properties that is easily manipulated and shaped but is not permanently stretched under tension.

The operating member may be formed from a variety of materials including Acrylonitrile Butadiene Styrene (ABS), nylon, or any other material exhibiting sufficient flexion / flexural modulus which has shape memory and which has shape memory to retain the ability to be repeatably compressed through manipulation by a user. Stainless steel arms, paired and joined or fused at one end, with opposed free ends, and optionally formed with milled gripping holes on the outer surfaces of the arms may be used. Optionally, steamed and formed plywood, impermeably coated with lacquer or a resin would be possible for use in some circumstances but brittle substances such as ceramic or glass would be unsuitable and soft substances such as a synthetic cellular foam or rubber would too flexible, and therefore not suitable for the operating member.

The operating member may be a compressible operating member having curved or angled arms movable towards each other under applied pressure and resiliently revertible when pressure is released, and in particular compressible when pressure is selectively applied to the operating member by the user. The operating member may be compressible sufficiently to move the distal ends of the arms towards each other in response to finger pressure.

The device may be provided as a part of a kit comprising the device described herein and a prosthetic graft comprising a tubular branch defining a lumen. In such a kit, the device may be provided "ready for use" in that the operating member and elongate flexible members are assembled such that the eye of the device surrounds the branch.

### Industrial Applicability

The device disclosed herein may be used in surgical procedures.

The device described herein provides a "fuss free" clip, which uses the same materials as in existing delivery systems, and can be used quickly and efficiently on both natural and synthetic vessels. It is also readily operated by a user intuitively due to its relative simplicity in comparison with several commercially available devices.

The device described herein may be advantageously used to provide haemostasis when different sized catheters are located in the vessel. The medical professional is not required to make any adjustment of the device when a catheter is inserted through the vessel which the device is controlling flow therethrough.

The device described herein provides a simple way for a medical professional to quickly selectively control the flow of fluid through a lumen by applying pressure to the operating member.

The device described herein provides loops which when tightened by pulling, create a smaller though hole or "eye". This interaction between loop and a surface of a vessel around which it is looped, for example a graft with a soft gelweave fabric surface, creates a conformable seal around any device inserted through a lumen in the graft, such as a catheter or sheathing around a device being delivered through the lumen of the graft.

Advantageously, the device may be disassembled and reassembled *in situ* by the medical professional if required.

The device described in this disclosure is applicable to both natural and synthetic vessels having a length and a transverse dimension defining a lumen or through passage, which vessels may be flexible walled vessels, allowing pinching or constriction applied thereto to close together pinched or constricted wall parts to reduce the transverse dimension of the lumen, additionally permitting closure upon an object such as a catheter within the lumen for example, to form a conformable seal therewith and thereby to completely occlude flow through the lumen when the catheter is in place or whilst it is being removed.

Reference numerals used in the above description of the accompanying drawings are also used in the claims simply for aiding identification of technical features of illustrated embodiments and increasing understanding of the claims without limitation of the scope thereof.

Modifications and improvements may be incorporated without departing from the scope of the invention, which is defined by the appended claims.

## Claims

1. A device (10; 40) for controlling fluid flow through a vessel (1) defining a lumen through which fluid may flow, comprising:
at least one elongate flexible member (12) configured to form an eye (20) for surrounding the vessel defining a lumen, the at least one elongate flexible member (12) having a first attachment point and a second attachment point; and
a compressible operating member (13; 43) attachable to the first attachment point and the second attachment point of said at least one elongate flexible member (12), the compressible operating member (13; 43) being compressible upon the application of pressure to the compressible operating member (13; 43), and operable to act upon the at least one elongate flexible member (12) such that: the at least one elongate flexible member (12) is movable from a first position in which the at least one elongate flexible member (12) is under tension such that the eye (20) constricts fluid flow within the lumen and a second position in which the at least one elongate flexible member (12) is relaxed such that the eye (20) does not constrict fluid flow through the lumen,
the compressible operating member (13; 43) is a C-shaped member having first and second arms (14, 16; 44, 46) separated by a gap (17; 47), wherein the first arm (14; 44) is attachable to the first attachment point of the at least one elongate flexible member (12), and the second arm (16; 46) is attachable to the second attachment point of the at least one elongate flexible member (12), wherein the eye (20) is located in the gap (17; 47), wherein at least one of the first and second arms (14, 16; 44, 46) is movable inwardly so as to move the at least one elongate flexible member (12) from the first position to the second position, **characterised in that**
the at least one elongate flexible member is a single elongate flexible member (12) comprising first and second ends (22, 24), wherein the first arm (14; 44) of the compressible operating member (13) comprises a first threading arrangement (26; 56), and the second arm (16; 46) of the compressible operating member (13) comprises a second threading arrangement (28; 58), wherein the first end (22) of the single elongate flexible member (12) is threaded through the first threading arrangement (26; 56) of the compressible operating member (13; 43), and the second end (24) of the single elongate flexible member (12) is threaded through the second threading arrangement (28; 58) of the compressible operating member (13; 43).

2. A device according to claim 1, wherein the compressible operating member (13) is operable to act on the single elongate flexible member (12) such that the single elongate flexible member (12) is biased towards the first position.

3. A device (40) according to of claim 1 or claim 2, wherein the C-shaped compressible operating member (43) has a proximal V- or U-shaped hinge portion (18; 48) having the first and second arms (14, 16; 44, 46) extending from the proximal V- or U-shaped hinge portion (18; 48), and each of the first and second arms (14, 16; 44, 46) has a distal free end for receiving the single elongate flexible member (12) for threading attachment of the single elongate flexible member (12) to the first and second arms (14, 16; 44, 46) of the operating member (13; 43).

4. The device (10) of claim 1, wherein at least one of the first and second threading arrangements (26, 28) comprises an aperture which is configured (30) to restrain a portion of the single flexible member (12) within the aperture by friction.

5. The device (40) of claim 4, wherein the aperture is a pinhole aperture (61) which has an internal surface (62, 63) which is roughened, or ribbed, or grooved, or serrated to increase frictional interaction with the single elongate flexible member (12).

6. The device (10) of claim 1, wherein each of the first and second threading arrangements (26, 28) comprises an aperture which is shaped so as to provide a plurality of winding points (30) for the threading of the single elongate flexible member.

7. A device (100; 40) for controlling fluid flow through a vessel (1; 101) defining a lumen through which fluid may flow, comprising:
at least one elongate flexible member (132, 134; 42, 42') configured to form an eye (120; 60) for surrounding the vessel defining a lumen, the at least one elongate flexible member (132, 134; 42, 42') having a first attachment point and a second attachment point; and
a compressible operating member (113; 43) attachable to the first attachment point and the second attachment point of said at least one elongate flexible member (132, 134; 42, 42'), wherein the compressible operating member (113; 43) has a proximal V- or U-shaped hinge portion (118; 48) having first and second arms (114, 116; 44, 46) separated by a gap (117; 47) extending from the proximal V- or U-shaped hinge portion (118; 48), and each of the first and second arms (114, 116; 44, 46) has a distal free end for receiving the at least one elongate flexible member (132, 134; 42, 42') for threading attachment of the at least one elongate flexible member (132, 134; 42, 42') to the first and second arms (114, 116; 44, 46) of the compressible operating member (113; 43), the compressible operating member (113; 43) being compressible upon the application of pressure to the compressible operating member (113; 43), and operable to act upon the at least one elongate flexible member (132, 134; 42, 42') such that: the at least one elongate flexible member (132, 134; 42, 42') is movable from a first position in which the at least one elongate flexible member (132, 134; 42, 42') is under tension such that the eye (120; 60) constricts fluid flow within the lumen and a second position in which the at least one elongate flexible member (132, 134; 42, 42') is relaxed such that the eye (20; 120; 60) does not constrict fluid flow through the lumen, and
the at least one elongate flexible member (132, 134; 42, 42') comprises: a first elongate flexible member (132; 42) configured to form a first loop (136); and a second elongate flexible member (134, 42') configured to form a second loop (138), wherein the first and second elongate flexible members (132, 134) are configured such that the first and second loops (136, 138) are linked so as to define the eye (120; 60) for surrounding the lumen, wherein a portion of the first elongate flexible member (132; 42) comprises the first attachment point and a portion of the second elongate flexible member (134; 42') comprises the second attachment point,
**characterised in that**
the first arm (114) comprises a first threading arrangement (126; 56), and the second arm(116) comprises a second threading arrangement (128; 58), wherein the first elongate flexible member (132, 42) is threaded through the first threading arrangement (126; 56) and second elongate flexible member (134; 42') is threaded through the second threading arrangement (128; 58).

8. A device (100; 40) according to claim 7, wherein the first and second elongate flexible members (132, 134; 42, 42') are configured such that the first and second loops (136, 138) intersect so as to define the eye (120; 60) for surrounding the lumen.

9. A device (10; 100; 40) according to any one of the preceding claims, wherein the at least one elongate flexible member (12; 132, 134; 42, 42') is formed from a resilient silicone material.

10. A device (10; 100; 40) according to any one of the preceding claims, wherein the at least one elongate flexible member (12; 132, 134; 42, 42') is a resilient cord or resilient filament.

11. A device (10; 100; 40) according to any one of the preceding claims, wherein the compressible operating member (13; 113; 43) is compressible upon the application of finger pressure, and the compressible operating member has upper surface and lower surface exterior indentations (344, 346) to facilitate finger pressure.

12. A kit for use in a surgical procedure comprising a device according to any one of claims 1 to 11 and a prosthetic graft comprising a tubular branch defining a lumen, wherein (a) the device is provided "ready for use" in that the compressible operating member and the at least one elongate flexible member of the device are assembled such that the eye of the device surrounds the tubular branch, or (b) the compressible operating member and the at least one elongate flexible member of the device are separate components to be assembled by a user.

## Patentansprüche

1. Eine Vorrichtung (10; 40) zum Steuern eines Fluidstroms durch ein Gefäß (1), das ein Lumen definiert, durch das Fluid strömen kann, die Folgendes beinhaltet:
mindestens ein längliches flexibles Element (12), das konfiguriert ist, um ein Auge (20) zum Umschließen des Gefäßes, das ein Lumen definiert, zu bilden, wobei das mindestens eine längliche flexible Element (12) einen ersten Befestigungspunkt und
einen zweiten Befestigungspunkt aufweist; und
ein komprimierbares Betätigungselement (13; 43), das an dem ersten Befestigungspunkt und dem zweiten Befestigungspunkt des mindestens einen länglichen flexiblen Elements (12) befestigt werden kann, wobei das komprimierbare Betätigungselement (13; 43) bei Aufbringen von Druck auf das komprimierbare Betätigungselement (13; 43) komprimierbar ist und dazu betreibbar ist, auf das mindestens eine längliche flexible Element (12) einzuwirken, sodass: das mindestens eine längliche flexible Element (12) von einer ersten Position, in der das mindestens eine längliche flexible Element (12) unter Spannung steht, sodass das Auge (20) den Fluidstrom innerhalb des Lumens einschränkt, in eine zweite Position, in der das mindestens eine längliche flexible Element (12) entspannt ist, sodass das Auge (20) den Fluidstrom durch das Lumen nicht einschränkt, beweglich ist,
das komprimierbare Betätigungselement (13; 43) ein C-förmiges Element ist, das einen ersten und einen zweiten Arm (14, 16; 44, 46) aufweist, die durch einen Spalt (17; 47) getrennt sind, wobei der erste Arm (14; 44) an dem ersten Befestigungspunkt des mindestens einen länglichen flexiblen Elements (12) befestigt werden kann und der zweite Arm (16; 46) an dem zweiten Befestigungspunkt des mindestens einen länglichen flexiblen Elements (12) befestigt werden kann, wobei sich das Auge (20) in dem Spalt (17; 47) befindet, wobei mindestens einer von dem ersten und dem zweiten Arm (14, 16; 44, 46) nach innen beweglich ist, um das mindestens eine längliche flexible Element (12) von der ersten Position in die zweite Position zu bewegen,
**dadurch gekennzeichnet, dass**
das mindestens eine längliche flexible Element ein einzelnes längliches flexibles Element (12) ist, das ein erstes und ein zweites Ende (22, 24) beinhaltet, wobei der erste Arm (14; 44) des komprimierbaren Betätigungselements (13) eine erste Fädelanordnung (26; 56) beinhaltet und der zweite Arm (16; 46) des komprimierbaren Betätigungselements (13) eine zweite Fädelanordnung (28; 58) beinhaltet, wobei das erste Ende (22) des einzelnen länglichen flexiblen Elements (12) durch die erste Fädelanordnung (26; 56) des komprimierbaren Betätigungselements (13; 43) gefädelt ist und das zweite Ende (24) des einzelnen länglichen flexiblen Elements (12) durch die zweite Fädelanordnung (28; 58) des komprimierbaren Betätigungselements (13; 43) gefädelt ist.

2. Vorrichtung gemäß Anspruch 1, wobei das komprimierbare Betätigungselement (13) dazu betreibbar ist, auf das einzelne längliche flexible Element (12) einzuwirken, sodass das einzelne längliche flexible Element (12) in Richtung der ersten Position vorgespannt ist.

3. Vorrichtung (40) gemäß Anspruch 1 oder Anspruch 2, wobei das C-förmige komprimierbare Betätigungselement (43) einen proximalen V- oder U-förmigen Gelenkabschnitt (18; 48) aufweist, wobei sich der erste und der zweite Arm (14, 16; 44, 46) von dem proximalen V- oder U-förmigen Gelenkabschnitt (18; 48) erstrecken, und jeder von dem ersten und dem zweiten Arm (14, 16; 44, 46) ein distales freies Ende zum Aufnehmen des einzelnen länglichen flexiblen Elements (12) zur Befestigung durch Hindurchfädeln des einzelnen länglichen flexiblen Elements (12) an dem ersten und dem zweiten Arm (14, 16; 44, 46) des Betätigungselements (13; 43) aufweist.

4. Vorrichtung (10) gemäß Anspruch 1, wobei mindestens eine von der ersten und zweiten Fädelanordnung (26, 28) eine Öffnung (30) beinhaltet, die konfiguriert ist, um einen Abschnitt des einzelnen flexiblen Elements (12) durch Reibung innerhalb der Öffnung zurückzuhalten.

5. Vorrichtung (40) gemäß Anspruch 4, wobei die Öffnung eine Nadellochöffnung (61) ist, die eine Innenfläche (62, 63) aufweist, die aufgeraut, oder gerippt, oder gerillt oder gezahnt ist, um die Reibungswechselwirkung mit dem einzelnen länglichen flexiblen Element (12) zu erhöhen.

6. Vorrichtung (10) gemäß Anspruch 1, wobei jede von der ersten und zweiten Fädelanordnung (26, 28) eine Öffnung beinhaltet, die so geformt ist, dass sie eine Vielzahl von Wickelpunkten (30) für das Fädeln des einzelnen länglichen flexiblen Elements bereitstellt.

7. Eine Vorrichtung (100; 40) zum Steuern eines Fluidstroms durch ein Gefäß (1; 101), das ein Lumen definiert, durch das Fluid strömen kann, die Folgendes beinhaltet:
mindestens ein längliches flexibles Element (132, 134; 42, 42'), das konfiguriert ist, um ein Auge (120; 60) zum Umschließen des Gefäßes, das ein Lumen definiert, zu bilden,
wobei das mindestens eine längliche flexible Element (132, 134; 42, 42') einen ersten Befestigungspunkt und einen zweiten Befestigungspunkt aufweist; und
ein komprimierbares Betätigungselement (113; 43), das an dem ersten Befestigungspunkt und dem zweiten Befestigungspunkt des mindestens einen länglichen flexiblen Elements (132, 134; 42, 42') befestigt werden kann, wobei das komprimierbare Betätigungselement (113; 43) einen proximalen V- oder U-förmigen Gelenkabschnitt (118; 48) aufweist, wobei sich ein erster und ein zweiter Arm (114, 116; 44, 46), die durch einen Spalt (117; 47) getrennt sind, von dem proximalen V- oder U-förmigen Gelenkabschnitt (118; 48) erstrecken, und jeder von dem ersten und dem zweiten Arm (114, 116; 44, 46) ein distales freies Ende zum Aufnehmen des mindestens einen länglichen flexiblen Elements (132, 134; 42, 42') zur Befestigung durch Hindurchfädeln des mindestens einen länglichen flexiblen Elements (132, 134; 42, 42') an dem ersten und dem zweiten Arm (114, 116; 44, 46) des komprimierbaren Betätigungselements (113; 43) aufweist, wobei das komprimierbare Betätigungselement (113; 43) bei Aufbringen von Druck auf das komprimierbare Betätigungselement (113; 43) komprimierbar ist und dazu betreibbar ist, auf das mindestens eine längliche flexible Element (132, 134; 42, 42') einzuwirken, sodass: das mindestens eine längliche flexible Element (132, 134; 42, 42') von einer ersten Position, in der das mindestens eine längliche flexible Element (132, 134; 42, 42') unter Spannung steht, sodass das Auge (120; 60) den Fluidstrom innerhalb des Lumens einschränkt, in eine zweite Position, in der das mindestens eine längliche flexible Element (132, 134; 42, 42') entspannt ist, sodass das Auge (20; 120; 60) den Fluidstrom durch das Lumen nicht einschränkt, beweglich ist, und
das mindestens eine längliche flexible Element (132, 134; 42, 42') Folgendes beinhaltet: ein erstes längliches flexibles Element (132; 42), das konfiguriert ist, um eine erste Schlaufe (136) zu bilden; und ein zweites längliches flexibles Element (134, 42'),
das konfiguriert ist, um eine zweite Schlaufe (138) zu bilden, wobei das erste und das zweite längliche flexible Element (132, 134) konfiguriert sind, sodass die erste und die zweite Schlaufe (136, 138) miteinander verbunden sind, um das Auge (120; 60) zum Umschließen des Lumens zu definieren, wobei ein Abschnitt des ersten länglichen flexiblen Elements (132; 42) den ersten Befestigungspunkt beinhaltet und ein Abschnitt des zweiten länglichen flexiblen Elements (134; 42') den zweiten Befestigungspunkt beinhaltet,
**dadurch gekennzeichnet, dass**
der erste Arm (114) eine erste Fädelanordnung (126; 56) beinhaltet und der zweite Arm (116) eine zweite Fädelanordnung (128; 58) beinhaltet, wobei das erste längliche flexible Element (132, 42) durch die erste Fädelanordnung (126; 56) gefädelt ist und
das zweite längliche flexible Element (134; 42') durch die zweite Fädelanordnung (128; 58) gefädelt ist.

8. Vorrichtung (100; 40) gemäß Anspruch 7, wobei das erste und das zweite längliche flexible Element (132, 134; 42, 42') konfiguriert sind, sodass sich die erste und die zweite Schlaufe (136, 138) schneiden, um das Auge (120; 60) zum Umschließen des Lumens zu definieren.

9. Vorrichtung (10; 100; 40) gemäß einem der vorhergehenden Ansprüche, wobei das mindestens eine längliche flexible Element (12; 132, 134; 42, 42') aus einem elastischen Silikonmaterial gebildet ist.

10. Vorrichtung (10; 100; 40) gemäß einem der vorhergehenden Ansprüche, wobei das mindestens eine längliche flexible Element (12; 132, 134; 42, 42') eine elastische Schnur oder ein elastisches Filament ist.

11. Vorrichtung (10; 100; 40) gemäß einem der vorhergehenden Ansprüche, wobei das komprimierbare Betätigungselement (13; 113; 43) bei Aufbringen von Fingerdruck komprimierbar ist und das komprimierbare Betätigungselement Außenvertiefungen (344, 346) an einer oberen Fläche und einer unteren Fläche aufweist, um Fingerdruck zu erleichtern.

12. Ein Kit zur Verwendung in einem chirurgischen Verfahren, das eine Vorrichtung gemäß einem der Ansprüche 1 bis 11 und ein prothetisches Transplantat beinhaltet, das eine röhrenförmige Abzweigung beinhaltet, die ein Lumen definiert, wobei (a) die Vorrichtung "gebrauchsfertig" bereitgestellt ist, wobei das komprimierbare Betätigungselement und das mindestens eine längliche flexible Element der Vorrichtung so zusammengesetzt sind, dass das Auge der Vorrichtung die röhrenförmige Abzweigung umschließt, oder (b) das komprimierbare Betätigungselement und das mindestens eine längliche flexible Element der Vorrichtung getrennte Komponenten sind, die von einem Benutzer zusammengesetzt werden.

## Revendications

1. Un dispositif (10 ; 40) pour contrôler un écoulement de fluide à travers un vaisseau (1) définissant une lumière à travers laquelle du fluide peut s'écouler, comprenant :
au moins un élément flexible allongé (12) configuré afin de former un œil (20) pour entourer le vaisseau définissant une lumière, l'au moins un élément flexible allongé (12) ayant un premier point de fixation et un deuxième point de fixation ; et
un élément d'actionnement compressible (13 ; 43) pouvant être fixé au premier point de fixation et au deuxième point de fixation dudit au moins un élément flexible allongé (12), l'élément d'actionnement compressible (13 ; 43) étant compressible lors de l'application d'une pression à l'élément d'actionnement compressible (13 ; 43), et pouvant être actionné afin d'agir sur l'au moins un élément flexible allongé (12) de telle sorte que :
l'au moins un élément flexible allongé (12) est mobile à partir d'une première position dans laquelle l'au moins un élément flexible allongé (12) est sous tension de telle sorte que l'œil (20) étrangle l'écoulement de fluide à l'intérieur de la lumière et d'une deuxième position dans laquelle l'au moins un élément flexible allongé (12) est relâché de telle sorte que l'œil (20) n'étrangle pas l'écoulement de fluide à travers la lumière,
l'élément d'actionnement compressible (13 ; 43) est un élément façonné en C ayant des premier et deuxième bras (14, 16 ; 44, 46) séparés par un écartement (17 ; 47), où le premier bras (14 ; 44) peut être fixé au premier point de fixation de l'au moins un élément flexible allongé (12), et le deuxième bras (16 ; 46) peut être fixé au deuxième point de fixation de l'au moins un élément flexible allongé (12), où l'œil (20) est situé dans l'écartement (17 ; 47), où au moins un des premier et deuxième bras (14, 16 ; 44, 46) est mobile vers l'intérieur de manière à déplacer l'au moins un élément flexible allongé (12) de la première position à la deuxième position, **caractérisé en ce que** l'au moins un élément flexible allongé est un élément flexible allongé unique (12) comprenant des première et deuxième extrémités (22, 24), où le premier bras (14 ; 44) de l'élément d'actionnement compressible (13) comprend un premier agencement d'enfilage (26 ; 56), et le deuxième bras (16 ; 46) de l'élément d'actionnement compressible (13) comprend un deuxième agencement d'enfilage (28 ; 58), où la première extrémité (22) de l'élément flexible allongé unique (12) est enfilée à travers le premier agencement d'enfilage (26 ; 56) de l'élément d'actionnement compressible (13 ; 43), et la deuxième extrémité (24) de l'élément flexible allongé unique (12) est enfilée à travers le deuxième agencement d'enfilage (28 ; 58) de l'élément d'actionnement compressible (13 ; 43).

2. Un dispositif selon la revendication 1, où l'élément d'actionnement compressible (13) peut être actionné afin d'agir sur l'élément flexible allongé unique (12) de telle sorte que l'élément flexible allongé unique (12) est sollicité vers la première position.

3. Un dispositif (40) selon la revendication 1 ou la revendication 2, où l'élément d'actionnement compressible façonné en C (43) a une portion de charnière proximale façonnée en V ou en U (18 ; 48) ayant les premier et deuxième bras (14, 16 ; 44, 46) qui s'étendent à partir de la portion de charnière proximale façonnée en V ou en U (18 ; 48), et chacun des premier et deuxième bras (14, 16 ; 44, 46) a une extrémité libre distale pour recevoir l'élément flexible allongé unique (12) pour la fixation par enfilage de l'élément flexible allongé unique (12) aux premier et deuxième bras (14, 16 ; 44, 46) de l'élément d'actionnement (13 ; 43).

4. Le dispositif (10) de la revendication 1, où au moins un des premier et deuxième agencements d'enfilage (26, 28) comprend une ouverture qui est configurée (30) pour retenir une portion de l'élément flexible unique (12) à l'intérieur de l'ouverture par frottement.

5. Le dispositif (40) de la revendication 4, où l'ouverture est une ouverture de trou d'épingle (61) qui a une surface interne (62, 63) qui est rendue rugueuse, ou nervurée, ou rainurée, ou dentelée afin d'augmenter une interaction de frottement avec l'élément flexible allongé unique (12).

6. Le dispositif (10) de la revendication 1, où chacun des premier et deuxième agencements d'enfilage (26, 28) comprend une ouverture qui est façonnée de manière à fournir une pluralité de points d'enroulement (30) pour l'enfilage de l'élément flexible allongé unique.

7. Un dispositif (100 ; 40) pour contrôler un écoulement de fluide à travers un vaisseau (1 ; 101) définissant une lumière à travers laquelle du fluide peut s'écouler, comprenant :
au moins un élément flexible allongé (132, 134 ; 42, 42') configuré afin de former un œil (120 ; 60) pour entourer le vaisseau définissant une lumière, l'au moins un élément flexible allongé (132, 134 ; 42, 42') ayant un premier point de fixation et un deuxième point de fixation ; et
un élément d'actionnement compressible (113 ; 43) pouvant être fixé au premier point de fixation et au deuxième point de fixation dudit au moins un élément flexible allongé (132, 134 ; 42, 42'), où l'élément d'actionnement compressible (113 ; 43) a une portion de charnière proximale façonnée en V ou en U (118 ; 48) ayant des premier et
deuxième bras (114, 116 ; 44, 46) séparés par un écartement (117 ; 47) qui s'étendent à partir de la portion de charnière proximale façonnée en V ou en U (118 ; 48), et chacun des premier et deuxième bras (114, 116 ; 44, 46) a une extrémité libre distale pour recevoir l'au moins un élément flexible allongé (132, 134 ; 42, 42') pour la fixation par enfilage de l'au moins un élément flexible allongé (132, 134 ; 42, 42') aux premier et deuxième bras (114, 116 ; 44, 46) de l'élément d'actionnement compressible (113 ; 43), l'élément d'actionnement compressible (113 ; 43) étant compressible lors de l'application d'une pression à l'élément d'actionnement compressible (113 ; 43), et pouvant être actionné afin d'agir sur l'au moins un élément flexible allongé (132, 134 ; 42, 42') de telle sorte que : l'au moins un élément flexible allongé (132, 134 ; 42, 42') est mobile à partir d'une première position dans laquelle l'au moins un élément flexible allongé (132, 134 ; 42, 42') est sous tension de telle sorte que l'œil (120 ; 60) étrangle l'écoulement de fluide à l'intérieur de la lumière et d'une deuxième position dans laquelle l'au moins un élément flexible allongé (132, 134 ; 42, 42') est relâché de telle sorte que l'œil (20 ; 120 ; 60) n'étrangle pas l'écoulement de fluide à travers la lumière, et
l'au moins un élément flexible allongé (132, 134 ; 42, 42') comprend : un premier élément flexible allongé (132 ; 42) configuré afin de former une première boucle (136) ; et un deuxième élément flexible allongé (134, 42') configuré afin de former une deuxième boucle (138), où les premier et deuxième éléments flexibles allongés (132, 134) sont configurés de telle sorte que les première et deuxième boucles (136, 138) sont liées de manière à définir l'œil (120 ; 60) pour entourer la lumière, où une portion du premier élément flexible allongé (132 ; 42) comprend le premier point de fixation et une portion du deuxième élément flexible allongé (134 ; 42') comprend le deuxième point de fixation,
**caractérisé en ce que**
le premier bras (114) comprend un premier agencement d'enfilage (126 ; 56), et le deuxième bras (116) comprend un deuxième agencement d'enfilage (128 ; 58), où le premier élément flexible allongé (132, 42) est enfilé à travers le premier agencement d'enfilage (126 ; 56) et le deuxième élément flexible allongé (134 ; 42') est enfilé à travers le deuxième agencement d'enfilage (128 ; 58).

8. Un dispositif (100 ; 40) selon la revendication 7, où les premier et deuxième éléments flexibles allongés (132, 134 ; 42, 42') sont configurés de telle sorte que les première et deuxième boucles (136, 138) se coupent de manière à définir l'œil (120 ; 60) pour entourer la lumière.

9. Un dispositif (10 ; 100 ; 40) selon l'une quelconque des revendications précédentes, où l'au moins un élément flexible allongé (12 ; 132, 134 ; 42, 42') est formé à partir d'un matériau silicone résilient.

10. Un dispositif (10 ; 100 ; 40) selon l'une quelconque des revendications précédentes, où l'au moins un élément flexible allongé (12 ; 132, 134 ; 42, 42') est un cordon résilient ou un filament résilient.

11. Un dispositif (10 ; 100 ; 40) selon l'une quelconque des revendications précédentes, où l'élément d'actionnement compressible (13 ; 113 ; 43) est compressible lors de l'application d'une pression digitale, et l'élément d'actionnement compressible a des indentations extérieures de surface supérieure et de surface inférieure (344, 346) afin de faciliter une pression digitale.

12. Un kit destiné à être utilisé dans une procédure chirurgicale comprenant un dispositif selon l'une quelconque des revendications 1 à 11 et un greffon prothétique comprenant une branche tubulaire définissant une lumière, où (a) le dispositif est fourni « prêt à l'emploi » en ce que l'élément d'actionnement compressible et l'au moins un élément flexible allongé du dispositif sont assemblés de telle sorte que l'œil du dispositif entoure la branche tubulaire, ou (b) l'élément d'actionnement compressible et l'au moins un élément flexible allongé du dispositif sont des composants séparés devant être assemblés par un utilisateur.
